(19) Europäisches Patentamt
European Patent Office
Office européen des brevets

(11) Veröffentlichungsnummer: **0 303 179**
**A1**

(12) # EUROPÄISCHE PATENTANMELDUNG

(21) Anmeldenummer: **88112675.9**

(22) Anmeldetag: **04.08.88**

(51) Int. Cl.⁴: **C07D 295/12 , C07D 333/20 , C07D 207/44 , C07D 317/66 , C07D 307/78 , C07D 317/58 , C07D 307/52 , A61K 31/40 , A61K 31/13 , C07C 93/02 , A61K 31/44**

(30) Priorität: **11.08.87 DE 3726633**

(43) Veröffentlichungstag der Anmeldung:
**15.02.89 Patentblatt 89/07**

(84) Benannte Vertragsstaaten:
**AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Leinert, Herbert, Dr. Ing.**
**Essigkamm 11**
**D-6148 Heppenheim(DE)**
Erfinder: **Tsaklakidis, Christos, Dr. phil. nat.**
**Weberstrasse 23**
**D-6940 Weinheim(DE)**
Erfinder: **Sponer, Gisbert, Dr. med. vet.**
**Lessingstrasse 13**
**D-6941 Laudenbach(DE)**

(54) **Neue 1,2-Diamino-Verbindungen, Verfahren zu ihrer Herstellung sowie Arzneimittel, die diese Verbindungen enthalten.**

(57) Verbindungen der Formel I

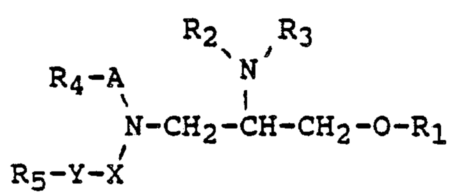

(I)

in der
$R_1$ einen gegebenenfalls substituierten Alkylrest, einen gegebenenfalls substituierten Alkenylrest, einen Cycloalkylrest oder einen gegebenenfalls substituierten mono- oder bicyclischen aromatischen Rest.
$R_2$ oder $R_3$ einen Alkylrest oder zusammen einen Ring,
A einen Valenzstrich oder einen Alkylenrest,
$R_4$ einen gegebenenfalls substituierten mono- oder bicyclischen aromatischen oder heteroaromatischen Rest,
X einen Valenzstrich oder einen Alkylenrest,
Y einen Valenzstrich oder ein Sauerstoffatom und
$R_5$ einen Cycloalkylrest oder einen gegebenenfalls substituierten mono- oder bicyclischen aromatischen

EP 0 303 179 A1

oder heteroaromatischen Rest bedeuten,
mit der Maßgabe, daß Y kein Sauerstoffatom darstellen darf, wenn X einen Valenzstrich bedeutet, und daß, für den Fall, daß $R_1$ einen gesättigten Kohlenwasserstoffrest darstellt, X einen Rest mit mindestens 2 C-Atomen haben muß, deren pharmakologischen verträgliche Salze, Verfahren zu ihrer Herstellung und Arzneimittel, die diese Verbindungen enthalten, zur Behandlung von Herz- und Kreislauferkrankungen.

# Neue 1,2-Diamino-Verbindungen, Verfahren zu ihrer Herstellung sowie Arzneimittel, die diese Verbindungen enthalten

Die vorliegende Erfindung betrifft neue 1,2-Diamino-Verbindungen, deren pharmakologisch verträgliche Salze, ihre Herstellung sowie diese enthaltende Arzneimittel.

Gegenstand der Erfindung sind neue 1,2-Diamino-Verbindungen der allgemeinen Formel I

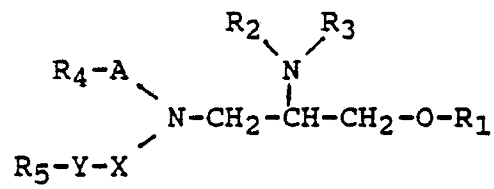

(I)

worin

$R_1$ einen geradkettigen oder verzweigten $C_1$-$C_{12}$ Alkylrest, der durch einen Phenyl-, Naphthyl- oder einen $C_3$-$C_7$-Cycloalkylrest substituiert sein kann,
einen geradkettigen oder verzweigten $C_2$-$C_6$-Alkenylrest, der durch einen $C_3$-$C_7$-Cycloalkylrest oder einen Phenyl- oder Naphthylrest substituiert sein kann,
einen $C_3$-$C_7$-Cycloalkylrest oder einen unsubstituierten oder einen ein- oder mehrfach substituierten mono- oder bicyclischen aromatischen Rest bedeutet,
wobei die Substituenten $C_1$-$C_4$ Alkyl, $C_1$-$C_4$ Alkoxy, Carboxyl oder Carbethoxy sein können,

$R_2$ und $R_3$ gleich oder verschieden sein können und einen geraden, verzweigten, gesättigten oder ungesättigten $C_1$-$C_6$-Alkylrest, der gegebenenfalls durch Hydroxy, $C_1$-$C_3$-Alkoxy oder $C_1$-$C_3$-Alkoxy-$C_1$-$C_3$-alkoxy substituiert sein kann, bedeuten oder zusammen mit dem Stickstoffatom einen gesättigten oder ungesättigten Ring bilden, der noch weitere Heteroatome enthalten kann und gegebenenfalls durch eine niedrige Alkylgruppe, eine niedrige Alkoxygruppe oder ein Sauerstoffatom substituiert ist,

A einen Valenzstrich oder einen geradkettigen oder verzweigten Alkylenrest mit 1-6 vorzugsweise 1-3 Kohlenstoffatomen bedeutet,

$R_4$ einen ein- oder mehrfach substituierten oder unsubstituierten mono- oder bicyclischen aromatischen oder heteroaromatischen Rest bedeutet,
wobei die Substituenten
Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-alkoxy-, Alkylamino-, Dialkylamino-, Alkoxy-carbonyl-alkoxy-, Alkylmercapto-, Alkylsulfinyl-, Alkylsufonyl-, Alkylsulfonyloxy-, Carboxy-, Alkoxy-carbonyl-, Amino-carbonyl-, Mono- oder Dialkylamino-carbonyl-, Halogenalkyl- oder Cyano-gruppen sowie Halogenatome, wie Chlor, Brom oder Fluor bedeuten.

X einen Valenzstrich oder einen geradkettigen, verzweigten, gesättigten oder ungesättigten Kohlenwasserstoffrest von 1 - 6 Kohlenstoffatomen bedeutet,

Y einen Valenzstrich oder ein Sauerstoffatom bedeutet,

und

$R_5$ einen $C_3$-$C_7$-Cycloalkyl-Rest oder einen ein- oder mehrfach substituierten oder einen unsubstituierten mono- oder bicyclischen aromatischen oder heteroaromatischen Rest bedeutet,
wobei die Substituenten
Alkyl-, Alkoxy-, $C_2$-$C_6$-Alkenyloxy-, Aralkoxy-, Hydroxy-, Hydroxy-alkoxy-, Alkoxy-alkoxy-, Alkoxy-carbonyl-alkyloxy-, $C_1$-$C_2$-Alkylendioxy-, Dialkylamino-, Alkylmercapto-, Alkylsulfinyl-, Alkylsulfonyl-, Alkylsulfonyloxy-, Hydroxy-alkyl-, Carboxy-, Alkoxycarbonyl-, Amino-carbonyl-, Mono- oder Dialkylamino-carbonyl- oder Cyano-gruppen sowie Halogenatome, wie Chlor, Brom oder Fluor bedeuten
mit der Maßgabe, daß Y kein Sauerstoffatom darstellen darf, wenn X einen Valenzstrich bedeutet und daß, in dem Fall, daß $R_1$ einen gesättigten Kohlenwasserstoffrest darstellt, X einen Rest mit mindestens zwei Kohlenstoffatomen bedeuten muß,
sowie deren pharmakologisch verträgliche Salze.

Der $C_1$-$C_{12}$-Alkylrest von $R_1$ bedeutet vorzugsweise Methyl, Ethyl, Propyl, Isopropyl, Isobutyl, Isoamyl, Isohexyl, n-Hexyl, n-Octyl, n-Dodecyl, insbesondere Isobutyl, Isoamyl und Isohexyl. Der $C_3$-$C_7$-Cycloalkylrest bedeutet in der Regel Cyclopentyl und Cyclohexyl. Wenn der Alkylrest substituiert ist, kommen insbesondere Cycloalkyl-methyl- und Benzyl- bzw. Phenethylgruppen in Frage. $C_2$-$C_6$-Alkenylreste sind vorzugsweise Allyl, Methallyl und Isopentenyle. Ein substituierter Rest stellt der Styryl- und Cinnamylrest dar. Mono- oder bicyclische aromatische Reste der Substituenten $R_1$, $R_4$ und $R_5$ sind vorzugsweise Phenyl,

Naphthyl, Indanyl, Indenyl und Tetralinyl.

$R_2$ und $R_3$ bedeuten vorzugsweise Methyl, Ethyl, Propyl, Allyl- oder Methallyl. Ringe, die $R_2$ und $R_3$ zusammen mit dem Stickstoffatom, an das sie gebunden sind, bilden können, sind vorzugsweise der Pyrrolidin- und der Piperidinring, insbesondere der Pyrrolidinring. Die Heteroatome, die die Ringe enthalten können, sind Stickstoff, Schwefel oder Sauerstoff.Es sind hierunter Ringe wie z. B. Piperazin, Morpholin und Thiomorpholin zu verstehen. Substituenten der vorstehend genannten Ringe sind insbesondere $C_1$-$C_3$ Alkyl- und $C_1$-$C_3$ Alkoxygruppen, wie z. B. Methyl, Ethyl oder Propyl oder Methoxy, Ethoxy oder Propoxy. Der Sauerstoff-Substituent stellt in der Regel mit dem C-Atom, an das er gebunden ist, eine Carbonylgruppe dar. Entsprechende Ringe sind z. B. der Pyrrolidinon- und Piperidinon-Ring.

Heteroaromatische Reste der Substituenten $R_4$ und $R_5$ sind vorzugsweise Pyridyl, Pyrimidinyl, Pyrazinyl, Thienyl, Oxazolyl, Pyrazolyl, Imidazolyl, Tetrazolyl, Thiazolyl, Isooxazolyl, Chinolinyl, Isochinolinyl, Benzofuranyl, Benzothienyl, Benzothiazolyl, Indolyl und Furanyl, insbesondere Benzofuranyl, Pyridyl, Furanyl, Thienyl.

Die Alkylgruppen, allein oder in Verbindung mit anderen Resten, von Substituenten von $R_4$ und $R_5$ der Ringsysteme enthalten 1-6, vorzugsweise 1-4 C-Atome und bedeuten insbesondere Methyl. Die Substitution kann ein- oder mehrfach sein.

Die erfindungsgemäßen Verbindungen der allgemeinen Formel I können in an sich bekannter Weise dargestellt werden, daß man

a) eine Verbindung der allgemeinen Formel II,

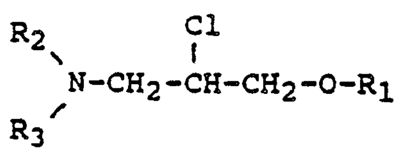

(II)

in der $R_1$, $R_2$, und $R_3$ die oben genannten Bedeutungen besitzen, mit einer Verbindung der allgemeinen Formel III,

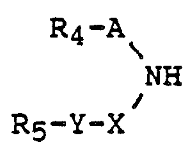

(III)

in der A, X, Y, $R_4$ und $R_5$ die oben genannten Bedeutungen besitzen, umsetzt

oder

b) eine Verbindung der allgemeinen Formel IV,

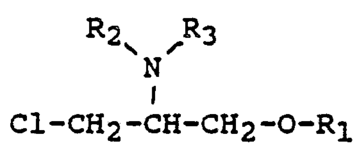

(IV)

in der $R_1$, $R_2$, und $R_3$ die oben genannten Bedeutungen besitzen, mit einer Verbindung der allgemeinen Formel III umsetzt,

oder

c) eine Verbindung der allgemeinen Formel V,

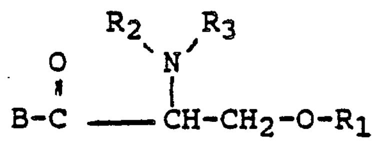

(V)

in der $R_1$, $R_2$, und $R_3$ die oben genannten Bedeutungen besitzen und B ein Halogenatom oder eine Alkoxygruppe bedeutet, einer Amidbildungsreaktion mit einer Verbindung der allgemeinen Formel III unterwirft und die erhaltene Verbindung der allgemeinen Formel VI,

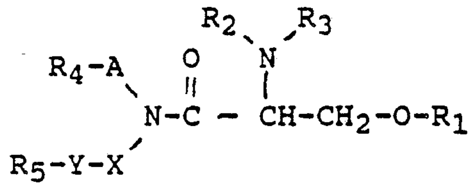

(VI)

in der $R_1$, $R_2$, $R_3$, $R_4$, $R_5$, A, X und Y die oben genannten Bedeutungen besitzen, einer Reduktion mit einem komplexen Hydrid oder Diboran unterzieht.

Die Umsetzung der Verbindungen der allgemeinen Formel II mit einer Verbindung der allgemeinen Formel III zu den erfindungsgemäßen Verbindungen der allgemeinen Formel I erfolgt, in an sich bekannter Weise, in einem inerten Lösungsmittel, wie Toluol, Xylol oder Dimethylformamid bei Temperaturen zwischen 40° C und Rückflußtemperatur des Lösungsmittels in Gegenwart eines alkalischen Kondensationsmittels, z.B. Natriumhydrid oder Natriumamid.

Die Verbindungen der allgemeinen Formel II können dargestellt werden, indem man eine Verbindung der allgemeinen Formel VII

$R_1OH$ (VII)

in der $R_1$ die angegebene Bedeutung besitzt mit Epichlorhydrin in Gegenwart von Natronlauge und eines Phasentransferkatalysators, z. B. Tetrabutylammoniumbromid zur Reaktion bringt und die erhaltene Verbindung der allgemeinen Formel VIII

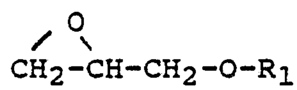

(VIII)

mit einem Amin der allgemeinen Formel IX

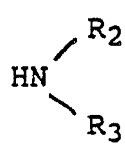

(IX)

in der $R_2$ und $R_3$ die angegebenen Bedeutungen besitzen, zur Reaktion bringt und die erhaltene Verbindung der allgemeinen Formel X

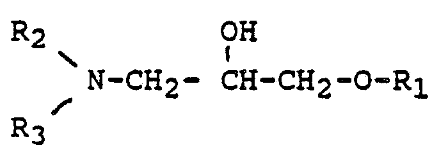

(X)

mit Thionylchlorid in einem inerten Lösungsmittel zu Verbindungen der allgemeinen Formel II umsetzt.

Die Umsetzung der Verbindungen der allgemeinen Formel IV mit Verbindungen der allgemeinen Formel III zu den erfindungsgemäßen Verbindungen der allgemeinen Formel I erfolgt in einem inerten Lösungsmittel wie Toluol oder Xylol bei Temperaturen zwischen 40° C und Rückflußtemperatur des Lösungsmittels in Gegenwart eines alkalischen Kondensationsmittels wie Natriumhydrid oder Natriumamid.

Die Verbindungen der allgemeinen Formel IV können dargestellt werden, daß man eine Verbindung der allgemeinen Formel XI

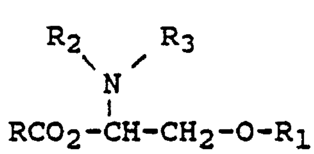

(XI)

in der $R_1$, $R_2$ und $R_3$ die angegebenen Bedeutungen besitzen und R einen Alkylrest bedeutet, mit einem komplexen Hydrid, z. B. Lithiumaluminiumhydrid in einem inerten Lösungsmittel in an sich bekannter Weise zu einer Verbindung der allgemeinen Formel XII

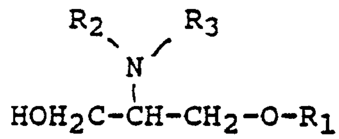

(XII)

reduziert und diese in einem inerten Lösungsmittel mit Thionylchlorid zu einer Verbindung der allgemeinen Formel IV umsetzt.

Die Ausgangsverbindungen der allgemeinen Formel XI können nach dem in der DE-B-2802864 beschriebenen Verfahren hergestellt werden.

Die Umsetzung der Verbindungen der allgemeinen Formel V mit einer Verbindung der allgemeinen Formel III zu einer Verbindung der allgemeinen Formel VI sowie die Reduktion dieser Verbindungen zu den erfindungsgemäßen Verbindungen der allgemeinen Formel I erfolgt nach Lit. bekannten Verfahren.

Die Verbindungen der allgemeinen Formel V, in der B ein Halogenatom bedeutet, können hergestellt werden, indem man eine Verbindung der allgemeinen Formel XI einer Hydrolyse unterwirft und die erhaltene Verbindung der allgemeinen Formel XIII

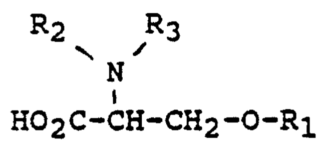

(XIII)

in der

$R_1$, $R_2$ und $R_3$ die oben angegebene Bedeutungen besitzen mit einem Halogenierungsmittel, z. B. Thionylchlorid in einem inerten Lösungsmittel zur Reaktion bringt.

Die Verbindungen der allgemeinen Formel III können dargestellt werden, indem man unter Beibehaltung der oben genannten Definitionen für A, X, Y, $R_4$ und $R_5$

α) eine Verbindung der allgemeinen Formel

$R_4A\text{-}NH_2$

mit einer Verbindung der allgemeinen Formel

$R_5\text{-}Y\text{-}COCl$

zur Reaktion bringt und das erhaltene Amid der allgemeinen Formel

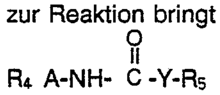

mit einem komplexen Hydrid oder Diboran reduziert oder

β) eine Verbindung der allgemeinen Formel

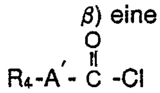

worin A′ ein Alkylrest mit 1 - 5 vorzugsweise 1 - 2 C-Atome bedeutet, mit einer Verbindung der allgemeinen Formel

$R_5\text{-}Y\text{-}X\text{-}NH_2$

umsetzt

und das erhaltene Amid der allgemeinen Formel

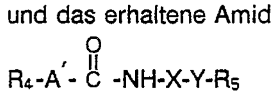

mit einem komplexen Hydrid oder Diboran reduziert.

oder

γ) durch eine reduktive Aminierung einer Carbonylverbindung der allgemeinen Formel

$$R_5\text{-Y-X}'\text{-}\overset{\displaystyle Z}{\overset{|}{C}}=O$$

wobei Z H oder Alkyl und X′ einen gesättigten, ungesättigten, geradkettigen oder verzweigten Alkylrest bedeutet, wobei X′ immer ein Kohlenstoffatom weniger enthält als X, mit einem Amin der allgemeinen Formel

$R_4ANH_2$

oder

δ) durch reduktive Aminierung einer Verbindung der allgemeinen Formel

$$R_4A'\text{-}\overset{\displaystyle V}{\overset{|}{C}}=O$$

wobei V Wasserstoff oder Alkyl bedeutet und A′ einen gesättigten oder ungesättigten, geradkettigen oder verzweigten Alkylrest darstellt, wobei A′ immer ein Kohlenstoffatom weniger enthält als A mit einer Verbindung der allgemeinen Formel

$R_5YX\text{-}NH_2$.

Die erfindungsgemäßen Verbindungen der allgemeinen Formel I besitzen ein asymmetrisches Kohlenstoffatom.

Gegenstand der Erfindung sind daher auch Racemate und die optisch aktiven Formen der erfindungsgemäßen Verbindungen der allgemeinen Formel I sowie Verfahren zu ihrer Herstellung.

Die optisch aktiven Verbindungen können aus ihren racemischen Mischungen nach an sich bekannten Methoden über diasteromere Salze hergestellt werden. Zur Racematenspaltung können z. B. Weinsäure, Apfelsäure, Camphersäure, Camphersulfonsäure oder Dibenzoylweinsäure verwendet werden.

Zur Überführung der Verbindungen der allgemeine Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Salicylsäure, Citronensäure, Benzoesäure, Naphthoesäure, o-Acetoxybenzoesäure, Adipinsäure, Maleinsäure, Oxalsäure, Fumarsäure oder Cyclaminsäure.

Die erfindungsgemäßen Verbindungen der allgemeinen Formel I besitzen wertvolle pharmakologische Eigenschaften. Sie zeichnen sich besonders durch eine gefäßrelaxierende Wirkung aus und können daher zur Therapie von Herz-/Kreislauf-Erkrankungen eingesetzt werden.

Die erfindungsgemäßen neuen Substanzen der allgemeinen Formel I und ihre Salze können in flüssiger oder fester Form enteral oder parenteral appliziert werden. Als Injektionsmedium kommt vorzugsweise Wasser zu Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxischen Salze), hochmolekulare Polymere (wie flüssiges Polyethylenoxyd) zur Viskositätsregulierung. Feste Trägerstoffe sind z.B. Stärke, Lactose, Mannit, Methylcellulose, Talkum hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole), für orale Applikation geeignete Zubereitungen können gewünschtenfalls Geschmacks- und Süßstoffe enthalten.

Neben den nachfolgenden aufgeführten Beispielen sind insbesondere die folgenden Verbindungen im Sinne der Anmeldung bevorzugt:

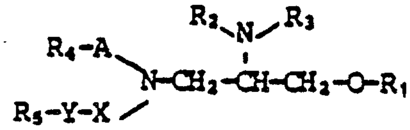

| $R_1$ | $R_2, R_3$ | $R_4$ | $R_5$ | A | X | Y |
|---|---|---|---|---|---|---|
| 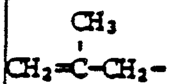 | 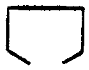 | 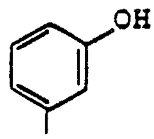 | 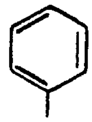 | - | $-CH_2-$ | - |
| 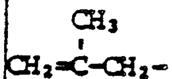 | 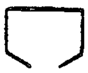 | 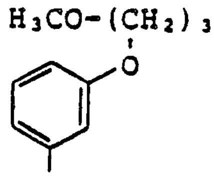 | 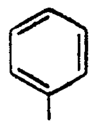 | - | $-CH_2-$ | - |
| 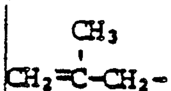 | 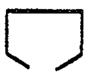 | 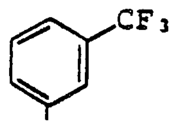 | 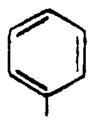 | - | $-CH_2-$ | - |
| 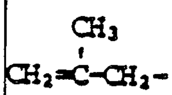 | 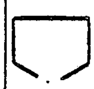 | 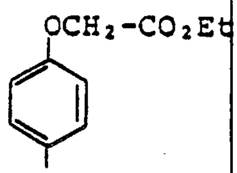 | 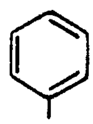 | - | $-CH_2-$ | - |
| 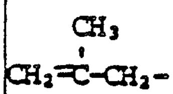 | 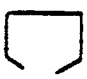 | 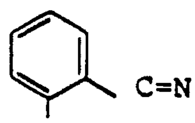 | 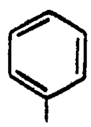 | - | $-CH_2-$ | - |

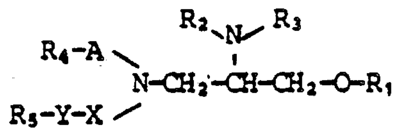

| $R_1$ | $R_2,R_3$ | $R_4$ | $R_5$ | A | X | Y |
|---|---|---|---|---|---|---|
| 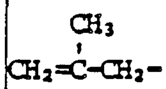 | 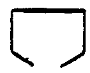 | 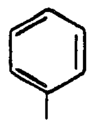 | 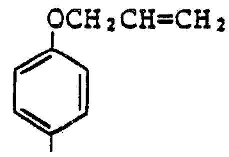 | - | $-CH_2-$ | - |
| 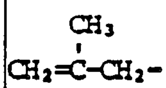 | 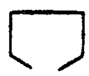 | 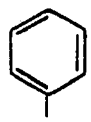 | 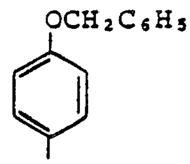 | - | $-CH_2-$ | - |
| 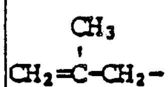 | 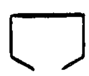 | 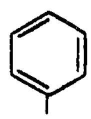 | 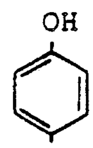 | - | $-CH_2-$ | - |
| 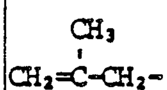 | 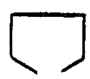 | 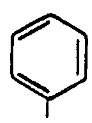 | 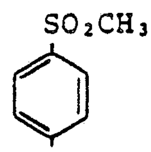 | - | $-CH_2-$ | - |
| 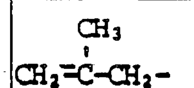 | 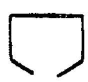 | 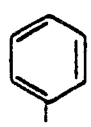 | 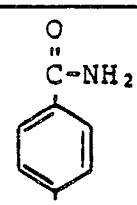 | - | $-CH_2-$ | - |
| 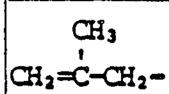 | 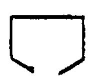 | 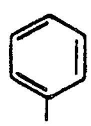 | 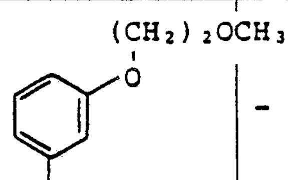 | - | $-CH_2-$ | - |
| 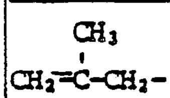 | 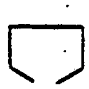 | 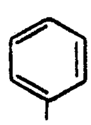 | 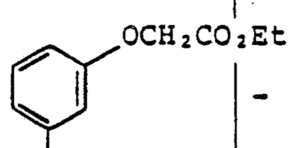 | - | $-CH_2-$ | - |

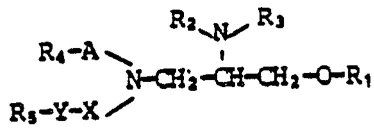

| $R_1$ | $R_2, R_3$ | $R_4$ | $R_5$ | A | X | Y |
|---|---|---|---|---|---|---|
| 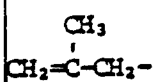 | 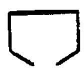 | 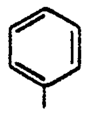 | 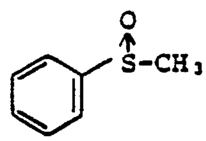 | - | $-CH_2-$ | - |
| 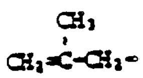 | $-CH_2-CH_2-OCH_3$<br>$-CH_2-CH_2-OCH_3$ | 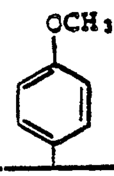 | 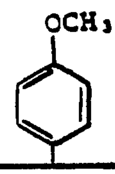 | - | $-CH_2-$ | - |
| 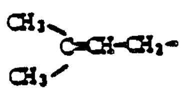 | $-(CH_2)_2-O-(CH_2)_2-OCH_3$<br>$-(CH_2)_2-O-(CH_2)_2-OCH_3$ | 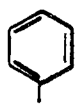 | 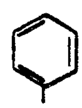 | - | $-CH_2-$ | - |
| 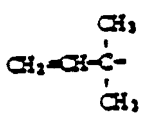 | $-CH_2-CH_2-OCH_3$<br>$-CH_2-CH_2-OCH_3$ | 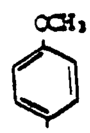 | 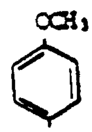 | - | $-CH_2-$ | - |
| 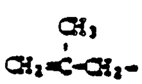 | $-CH_2-CH_2-OCH_3$<br>$-CH_2-CH_2-OCH_3$ | 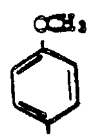 | 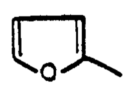 | - | $-CH_2-$ | - |
| 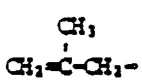 | $-CH_2-CH_2-OCH_3$<br>$-CH_2-CH_2-OCH_3$ | 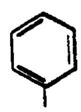 | 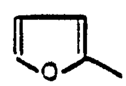 | - | $-CH_2-$ | - |

Beispiel 1:

2-(N-Pyrrolidino)-3-isobutoxy-N-phenyl-N-(2-phenyl-ethyl)-propylamin-Oxalat

6.3 g N-Phenyl-N-(2-phenyl-ethyl)-amin werden zusammen mit 7.7 g 2-Chlor-1-isobutoxy-3-(N-pyrrolidino)-propan in 50 ml absol. Toluol gelöst. Hierzu gibt man 5.8 g Natriumhydrid (50proz. ölige Suspension) und erhitzt die Mischung 2 Stdn. am Rückfluß. Man kühlt ab, gibt die Mischung auf Wasser und trennt die organische Schicht ab. Die Wasserphase wird nochmals mit Toluol extrahiert. Die vereinigten organischen Phasen werden mit Wasser gewaschen, über Natriumsulfat getrocknet und eingedampft. Der Rückstand wird durch Chromatographie an einer Kieselgelsäule gereinigt. (Fließmittel: Methylenchlorid/2 % Methanol).

Die entsprechenden Säulenfranktionen werden eingedampft. Der Rückstand wird in Essigester gelöst und die Lösung mit einer Lösung von Oxalsäure in Essigester versetzt. Der Niederschlag wird abfiltriert und nochmals aus Essigester umkristallisiert. Man erhält 2.1 g 2-(N-Pyrrolidino)-3-isobutoxy-N-phenyl-N-(2-phenyl-ethyl)-propylamin-Oxalat vom Schmp.: 95 - 96° C.

Die als Ausgangsmaterialien benötigten Halogen-Verbindungen der allgemeinen Formel II wurden analog dem als repräsentativem Beispiel beschriebenen 2-Chlor-1-allyloxy-3-N-pyrrolidino-propan dargestellt.

Eine Mischung von 50 g Allylalkohol, 160 ml konz. Natronlauge, 230 g Epichlorhydrin und 2 g Tetrabutylammonium-bromid wird 3 Stdn. bei 45° C gerührt. Dann wird die Mischung mit Wasser verdünnt und mit Essigester extrahiert. Die organische Phase wird mit Wasser gewaschen, über Natriumsulfat getrocknet und eingedampft. Der Rückstand wird i. Vak. destilliert. Man erhält 67 g 3-Allyloxy-1,2-epoxypropan vom Sdp.: 78° C/56 mm. Dieses wird in 80 ml Ethanol gelöst und unter Rühren mit einer Lösung von 86 ml Pyrrolidin in 80 ml Ethanol tropfenweise versetzt. Nach beendeter Zugabe rührt man noch 1 Std. bei 70° C weiter, dampft ein und destilliert den Rückstand i. Vak.. Man erhält 70 g 2-Hydroxy-1-allyloxy-3-N-pyrrolidino-propan. Sdp.: 75° C/$10^{-2}$ mm.

60 g 2-Hydroxy-1-allyloxy-3-N-pyrrolidino-propan werden in 120 ml Dichlorethan gelöst und die Lösung tropfenweise unter Rühren mit 26 ml Thionylchlorid versetzt. Nach beendeter Zugabe rührt man noch 2 Stdn. bei 70° C weiter, kühlt ab, versetzt die Lösung mit Wasser, stellt den pH-Wert mit Natronlauge auf pH 10 ein und trennt die org. Phase ab. Diese wird über Natriumsulfat getrocknet und eingedampft. Der Rückstand wird durch Säulenchromatographie gereinigt. Man erhält 44 g 2-Chlor-1-allyloxy-3-N-pyrrolidino-propan als öliges Produkt.

Die folgenden Beispiele wurden analog dargestellt.

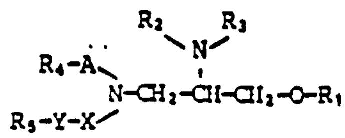

| Nr. | $R_1$ | $R_2,R_3$ | $R_4$ | $R_5$ | A | X | Y | |
|---|---|---|---|---|---|---|---|---|
| 2 | $(CH_3)_2$-CH-$CH_2$ - | 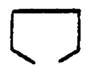 | 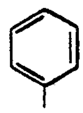 | 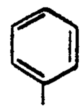 | - | -$(CH_2)_2$- | - | Oxalat 95 - 96° C Essigester |
| 3 | $(CH_3)_2$-CH-$CH_2$ - | 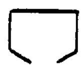 | 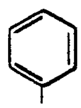 | 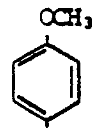 | - | -$(CH_2)_2$- | - | Fumarat 103 - 104° C Essigester |
| 4 | $(CH_3)_2$-CH-$CH_2$- | 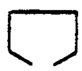 | 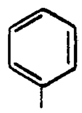 | 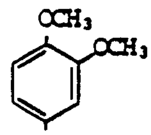 | - | -$(CH_2)_2$- | - | Oxalat 147 - 148° C Essigester |
| 5 | $(CH_3)_2$-CH-$CH_2$- | 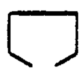 | 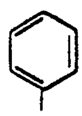 | 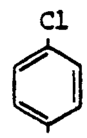 | - | -$(CH_2)_2$- | - | Oxalat 134° C Essigester |
| 6 | $(CH_3)_2$-CH-$CH_2$- | 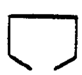 | 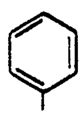 | 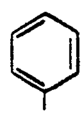 | - | -$(CH_2)_2$- | O | Fumarat 94 - 96° C Essigester |
| 7. | $(CH_3)_2$-CH-$CH_2$- | 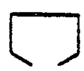 | 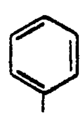 | 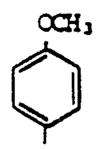 | - | -$(CH_2)_2$- | O | Fumarat 134° C Essigester |
| 8 | $(CH_3)_2$-CH-$CH_2$- | 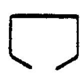 | 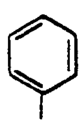 | 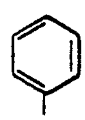 | - | -$(CH_2)_3$- | - | Oxalat 96 - 97° C Essigester |
| 9 | $(CH_3)_2$-CH-$CH_2$- | 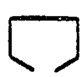 | 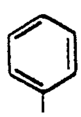 | 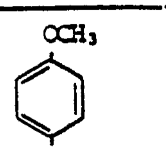 | - | -$(CH_2)_3$- | - | Oxalat 112 - 113° C Essigester |

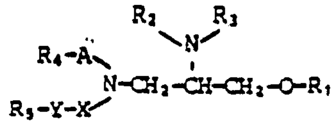

| Nr. | $R_1$ | $R_2$, $R_3$ | $R_4$ | $R_5$ | A | X | Y | |
|---|---|---|---|---|---|---|---|---|
| 10 | $(CH_3)_2-CH-CH_2-$ | 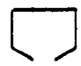 | 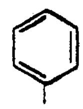 | 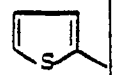 | - | $-(CH_2)_2-$ | - | Oxalat<br>95 - 96° C<br>Essigester |
| 11 | $(CH_3)_2-CH-CH_2-$ | 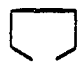 | 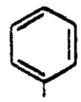 | 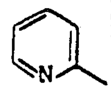 | - | $-(CH_2)_2-$ | - | Öl<br>m/e 380 |
| 12 | $(CH_3)_2-CH-CH_2-$ | 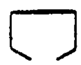 | 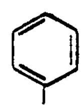 | 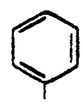 | $CH_2-$ | $-(CH_2)_2-$ | - | Öl<br>m/e 394 |
| 13 | $(CH_3)_2-CH-CH_2-$ | 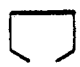 | 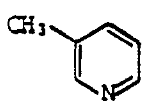 | 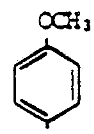 | - | $-(CH_2)_2-$ | - | Öl<br>m/e 425 |
| 14 | $CH_2=CH-CH_2-$ | 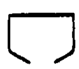 | 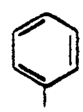 | 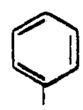 | - | $-CH_2-$ | - | Oxalat<br>152 - 153° C<br>Essigester |
| 15 | $CH_3$<br>$CH_2=C-CH_2-$ | 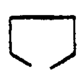 | 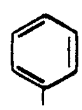 | 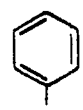 | - | $-CH_2-$ | - | Oxalat<br>159 - 161° C<br>Isopropanol |
| 16 | $CH_3$<br>$CH_2=C-CH_2-$ | 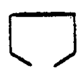 | 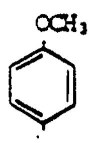 | 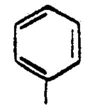 | - | $-CH_2-$ | - | Oxalat<br>128 - 130° C<br>Essigester |
| 17 | $CH_3$<br>$CH_2=C-CH_2-$ | 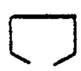 | 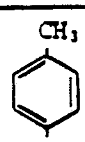 | 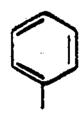 | - | $-CH_2-$ | - | Oxalat<br>152° C<br>Essigester |

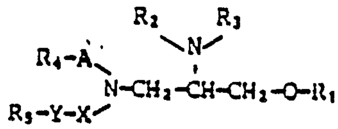

| Nr. | $R_1$ | $R_2,R_3$ | $R_4$ | $R_5$ | A | X | Y | |
|---|---|---|---|---|---|---|---|---|
| 18 | $CH_3$<br>$CH_2=C-CH_2-$ | 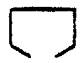 | 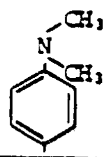 | 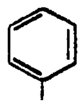 | - | $-CH_2-$ | - | Öl<br>m/e 407 |
| 19 | $CH_3$<br>$CH_2=C-CH_2-$ | 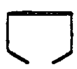 | 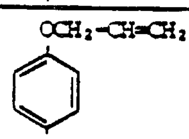 | 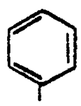 | - | $-CH_2-$ | - | Öl<br>m/e 420 |
| 20 | $CH_3$<br>$CH_2=C-CH_2-$ | 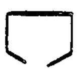 | 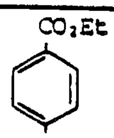 | 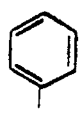 | - | $-CH_2-$ | - | Öl<br>m/e 436 |
| 21 | $CH_3$<br>$CH_2=C-CH_2-$ | 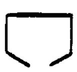 | 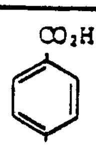 | 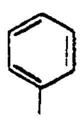 | - | $-CH_2-$ | - | Essigester<br>159° C |
| 22 | $CH_3$<br>$CH_2=C-CH_2-$ | 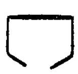 | 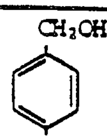 | 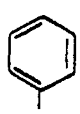 | - | $-CH_2-$ | - | Öl<br>m/e 394 |
| 23 | $CH_3$<br>$CH_2=C-CH_2-$ | 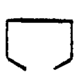 | 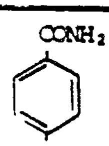 | 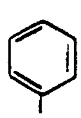 | - | $-CH_2-$ | - | Öl<br>m/e 407 |
| 24 | $CH_3$<br>$CH_2=C-CH_2-$ | 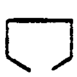 | 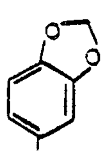 | 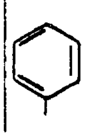 | - | $-CH_2-$ | - | Oxalat<br>125 - 126 °<br>Essigester |

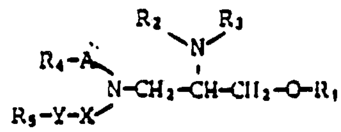

| Nr. | $R_1$ | $R_2,R_3$ | $R_4$ | $R_5$ | A | X | Y | |
|---|---|---|---|---|---|---|---|---|
| 25 | $CH_3$<br>$CH_2=C-CH_2-$ | 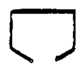 | 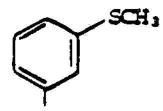 | 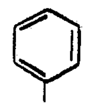 | - | $-CH_2-$ | - | Oxalat<br>114 - 115 °C<br>Essigester |
| 26 | $CH_3$<br>$CH_2=C-CH_2-$ | 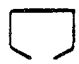 | 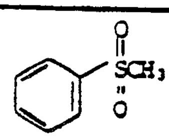 | 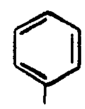 | - | $-CH_2-$ | - | Öl<br>m/e 442 |
| 27 | $CH_3$<br>$CH_2=C-CH_2-$ | 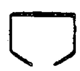 | 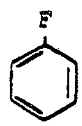 | 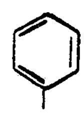 | - | $-CH_2-$ | - | Oxalat<br>173 - 174 °C<br>Essigester |
| 28 | $CH_3$<br>$CH_2=C-CH_2-$ | 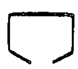 | 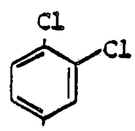 | 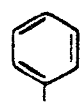 | - | $-CH_2-$ | - | Oxalat<br>192 - 193 °C<br>Essigester |
| 29 | $CH_3$<br>$CH_2=C-CH_2-$ | 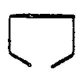 | 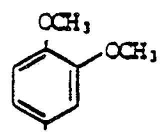 | 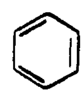 | - | $-CH_2-$ | - | Öl<br>m/e 424 |
| 30 | $CH_3$<br>$CH_2=C-CH_2-$ | 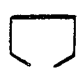 | 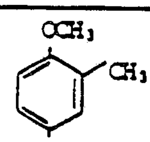 | 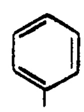 | - | $-CH_2-$ | - | Öl<br>m/e 408 |
| 31 | $CH_3$<br>$CH_2=C-CH_2-$ | 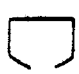 | 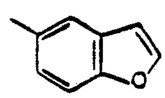 | 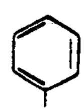 | - | $-CH_2-$ | - | Oxalat<br>135 - 136 °C<br>Isopropanol |
| 32 | $CH_3$<br>$CH_2=C-CH_2-$ | 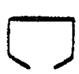 | 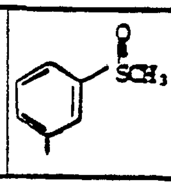 | 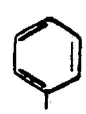 | - | $-CH_2-$ | - | Öl<br>m/e 426 |

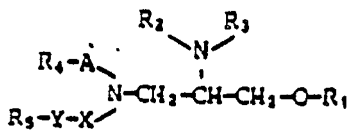

| Nr. | $R_1$ | $R_2,R_3$ | $R_4$ | $R_5$ | A | X | Y | |
|---|---|---|---|---|---|---|---|---|
| 33 | 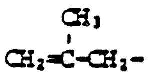 | 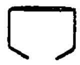 | 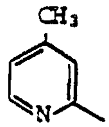 | 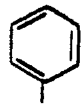 | - | $-CH_2-$ | - | Öl<br>m/e 411 |
| 34 | 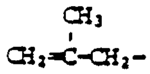 | 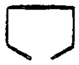 | 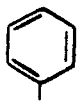 | 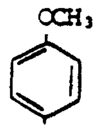 | - | $-CH_2-$ | - | Oxalat<br>128 - 130° C<br>Essigester |
| 35 | 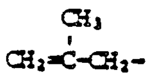 | 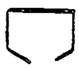 | 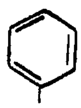 | 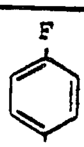 | - | $-CH_2-$ | - | Öl<br>m/e 382 |
| 36 | 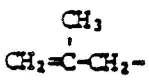 | 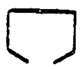 | 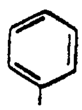 | 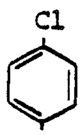 | - | $-CH_2-$ | - | Öl<br>m/e 398 |
| 37 | 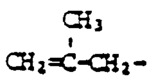 | 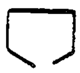 | 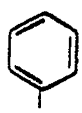 | 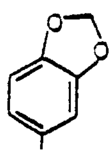 | - | $-CH_2-$ | - | Öl<br>m/e 408 |
| 38 | 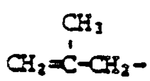 | 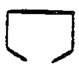 | 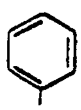 | 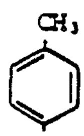 | - | $-CH_2-$ | - | Öl<br>m/e 378 |
| 39 | 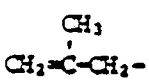 | 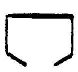 | 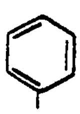 | 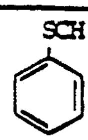 | - | $-CH_2-$ | - | Öl<br>m/e 410 |
| 40 | 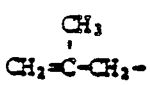 | 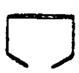 | 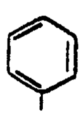 | 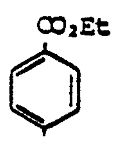 | - | $-CH_2-$ | - | Öl<br>m/e 436 |

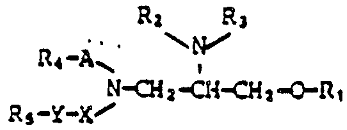

| Nr. | $R_1$ | $R_2,R_3$ | $R_4$ | $R_5$ | A | X | Y | |
|---|---|---|---|---|---|---|---|---|
| 41 | $CH_3$<br>$CH_2=C-CH_2-$ | 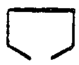 | 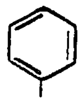 | 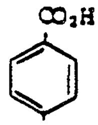 | - | $-CH_2-$ | - | Öl<br>m/e 408 |
| 42 | $CH_3$<br>$CH_2=C-CH_2-$ | 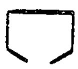 | 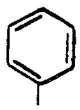 | 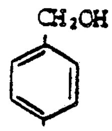 | - | $-CH_2-$ | - | Öl<br>m/e 394 |
| 43 | $CH_3$<br>$CH_2=C-CH_2-$ | 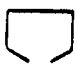 | 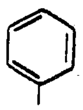 | 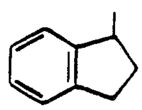 | - | - | - | Öl<br>m/e 390 |
| 44 | $CH_3$<br>$CH_2=C-CH_2-$ | 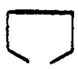 | 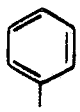 | 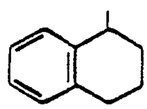 | - | - | - | Öl<br>m/e 402 |
| 45 | $CH_3$<br>$CH_2=C-CH_2-$ | 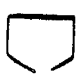 | 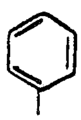 | 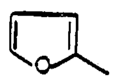 | - | $-CH_2-$ | - | Oxalat<br>130 - 131° C<br>Essigester |
| 46 | $CH_3$<br>$CH_2=C-CH_2-$ | 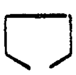 | 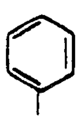 | 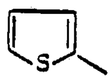 | - | $-CH_2-$ | - | Oxalat<br>135 - 136° C<br>Essigester |
| 47 | $CH_3$<br>$CH_2=C-CH_2-$ | 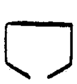 | 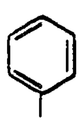 | 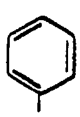 | - | $-(CH_2)_2-$ | - | Oxalat<br>79° C<br>Essigester |

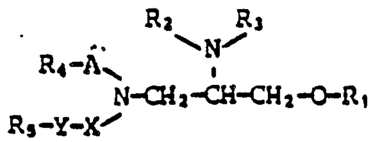

| Nr. | R₁ | R₂,R₃ | R₄ | R₅ | A | X | Y | |
|---|---|---|---|---|---|---|---|---|
| 48 | 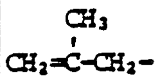 | 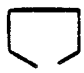 | 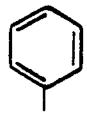 | 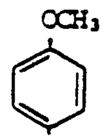 | - | $-(CH_2)_2-$ | - | Oxalat<br>125 - 126° C<br>Essigester |
| 49 | 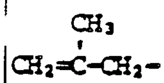 | 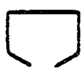 | 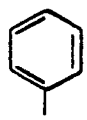 | 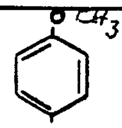 | - | $-(CH_2)_3$ | - | Fumarat<br>127° C<br>Essigester |
| 50 | 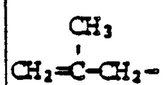 | 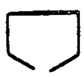 | 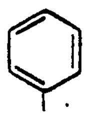 | 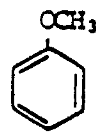 | - | $-(CH_2)_2$ | O | Oxalat<br>118 - 119° C<br>Essigester |
| 51 | 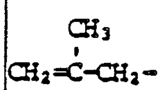 | 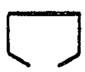 | 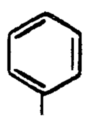 | 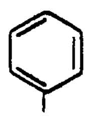 | - | $-(CH_2)_3$ | - | Oxalat<br>130 - 131°<br>Essigester |
| 52 | 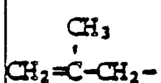 | 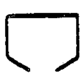 | 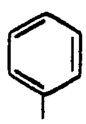 | 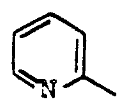 | - | $-CH_2-$ | - | Öl<br>m/e 365 |
| 53 | 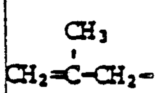 | 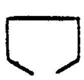 | 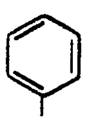 | 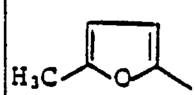 | - | $-CH_2-$ | - | Öl<br>m/e 368 |
| 54 | 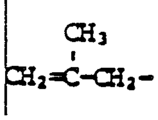 | 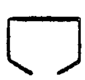 | 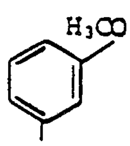 | 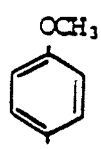 | - | $-CH_2-$ | - | Öl<br>m/e 424 |
| 55 | 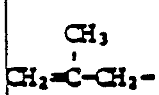 | 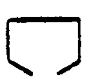 | 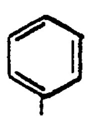 | 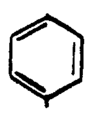 | - | $-(CH_2)_2$ | O | Oxalat<br>118 - 120°C |

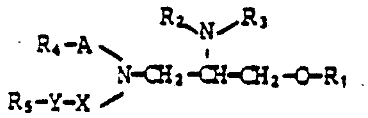

| Nr. | $R_1$ | $R_2, R_3$ | $R_4$ | $R_5$ | A | X | Y | |
|---|---|---|---|---|---|---|---|---|
| 56 | $CH_3$<br>$CH_2=C-CH_2-$ | 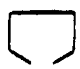 | 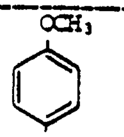 | 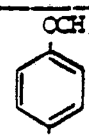 | - | $-CH_2-$ | - | Öl<br>m/e 424 |
| 57 | $CH_3$<br>$CH_2=C-CH_2-$ | 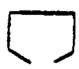 | 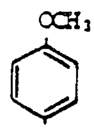 | 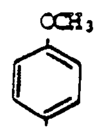 | - | $-(CH_2)_2-$ | - | Oxalat<br>97-98° C<br>Essigester |
| 58 | $CH_3$<br>$CH_2=C-CH_2-$ | 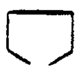 | 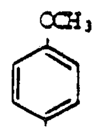 | 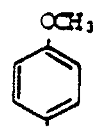 | - | $-(CH_2)_2-$ | O | Oxalat<br>93-95° C<br>Essigester |
| 59 | $CH_3$<br>$CH_2=C-CH_2-$ | 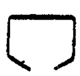 | 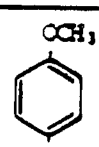 | 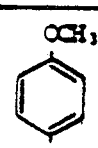 | - | $-(CH_2)_3-$ | - | Öl<br>m/e 452 |
| 60 | $CH_3$<br>$CH_2=C-CH_2-$ | 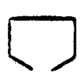 | 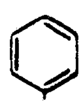 | 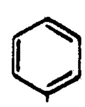 | - | $-(CH_2)_4-$ | - | Oxalat<br>92-95° C |

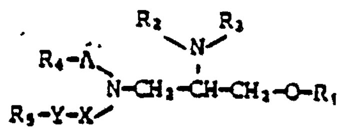

| Nr. | $R_1$ | $R_2,R_3$ | $R_4$ | $R_5$ | A | X | Y | |
|---|---|---|---|---|---|---|---|---|
| 61 | $CH_3$<br>$CH_2=C-CH_2-$ | 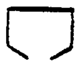 | 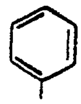 | 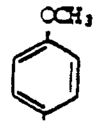 | $-CH_2-$ | $-CH_2-$ | - | Öl<br>m/e 408 |
| 62 | $CH_3$<br>$CH_2=C-CH_2-$ | 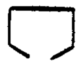 | 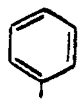 | 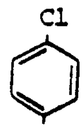 | $-CH_2-$ | $-CH_2-$ | - | Öl<br>m/e 413 |
| 63 | $CH_3$<br>$CH_2=C-CH_2-$ | 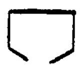 | 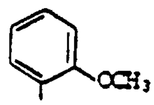 | 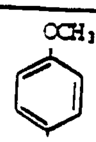 | - | $-CH_2-$ | - | Öl<br>m/e 424 |
| 64 | $CH_3$<br>$CH_2=C-CH_2-$ | 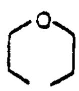 | 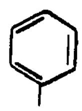 | 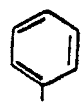 | - | $-CH_2-$ | - | Öl<br>m/e 380 |
| 65 | $CH_3$<br>$CH_2=C-CH_2-$ | 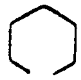 | 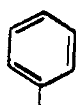 | 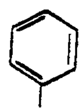 | - | $-CH_2-$ | - | Öl<br>m/e 378 |
| 66 | $CH_3$<br>$CH_2=C-CH_2-$ | $-C_2H_5$<br>$-C_2H_5$ | 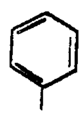 | 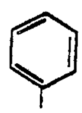 | - | $-CH_2-$ | - | Oxalat<br>61 - 63° C<br>Essigester |
| 67 | $CH_3$<br>$C=CH-CH_2-$<br>$CH_3$ | 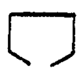 | 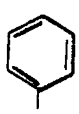 | 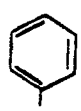 | - | $-CH_2-$ | - | Oxalat<br>149 - 150°C<br>Essigester |
| 68 | $CH_3$<br>$CH_2=C-CH_2-$ | 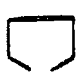 | 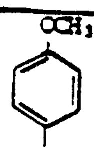 | 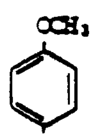 | $-CH_2-$ | $-CH_2-$ | - | Öl<br>m/e 438 |

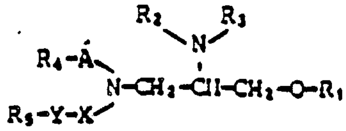

| Nr. | $R_1$ | $R_2,R_3$ | $R_4$ | $R_5$ | A | X | Y | |
|---|---|---|---|---|---|---|---|---|
| 69 | 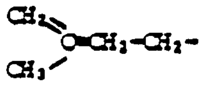 | 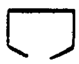 | 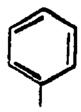 | 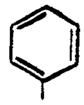 | - | $-CH_2-$ | - | Oxalat 151-152°C Essigester |
| 70 | 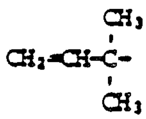 | 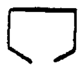 | 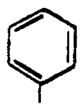 | 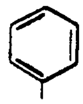 | - | $-CH_2-$ | - | Öl m/e 378 |
| 71 | $(CH_3)_2-CH-CH_2-$ | 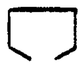 | 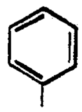 | 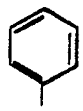 | - | $-CH=CHCH_2-$ | - | Oxalat 119-120°C Essigester |
| 72 | $(CH_3)_2-CH-CH_2-$ | 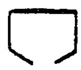 | 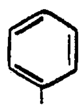 | 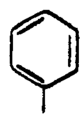 | - | 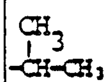 | - | Öl m/e 380 |
| 73 | 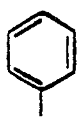 | 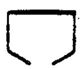 | 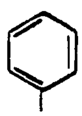 | 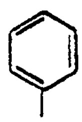 | - | $-CH_2-$ | - | Oxalat 136-137°C Essigester |
| 74 | 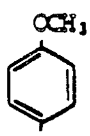 | 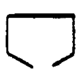 | 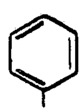 | 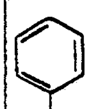 | - | $-CH_2-$ | - | Öl m/e 416 |
| 75 | 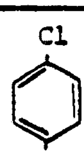 | 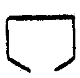 | 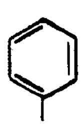 | 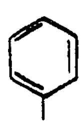 | - | $-CH_2-$ | - | Öl m/e 421 |
| 76 | 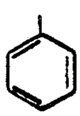 | 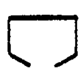 | 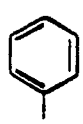 | 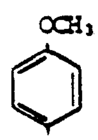 | - | $-(CH_2)_2-$ | - | Öl m/e 430 |

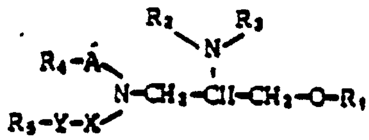

| Nr. | $R_1$ | $R_2,R_3$ | $R_4$ | $R_5$ | A | X | Y | |
|---|---|---|---|---|---|---|---|---|
| 77 | $(CH_3)_2-CH-CH_2-$ | 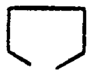 | 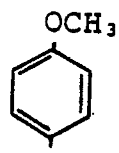 | 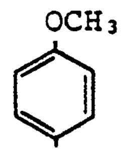 | - | $-(CH_2)_2-$ | - | Öl m/e 440 |
| 78 | $(CH_3)_2-CH-CH_2-$ | 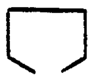 | 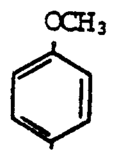 | 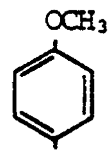 | - | $-(CH_2)_2-$ | 0 | Öl m/e 456 |
| 79 | $(CH_3)_2-CH-CH_2-$ | 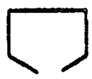 | 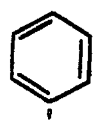 | 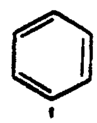 | - | $-(CH_2)_4-$ | - | Öl m/e 408 |
| 80 | $(CH_3)_2-CH-CH_2-$ | 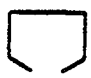 | 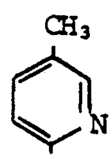 | 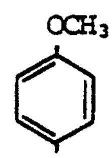 | - | $-CH_2-$ | - | Öl m/e 411 |
| 81 | 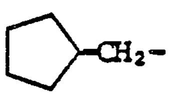 | 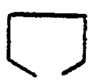 | 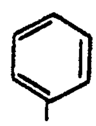 | 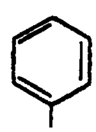 | - | $-CH_2-$ | - | Fumarat 137-138°C Essigester |
| 82 | 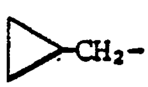 | 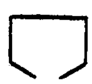 | 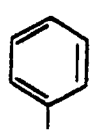 | 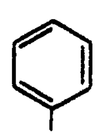 | - | $-CH_2-$ | - | Öl m/e 364 |

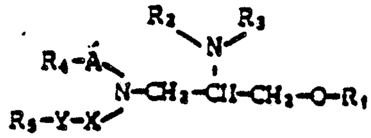

| Nr. | $R_1$ | $R_2,R_3$ | $R_4$ | $R_5$ | A | X | Y | |
|---|---|---|---|---|---|---|---|---|
| 83 | 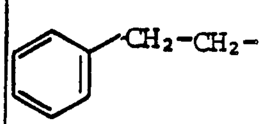 | 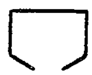 | 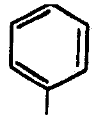 | 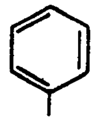 | - | $-CH_2-$ | - | Oxalat<br>133-135°C<br>Essigester |
| 84 | 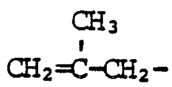 | 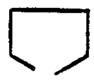 | 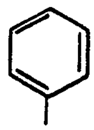 | 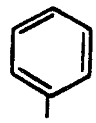 | - | - | - | Oxalat<br>105° C<br>Essigester |
| 85 | 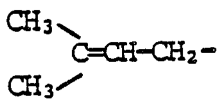 | 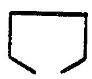 | 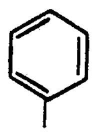 | 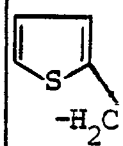 | - | $-CH_2-$ | - | Oxalat<br>118° C<br>Essigester |
| 86 | 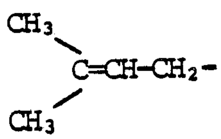 | 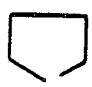 | 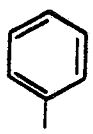 | 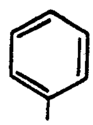 | - | - | - | Oxalat<br>120° C<br>Essigester |
| 87 | 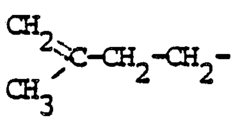 | 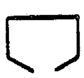 | 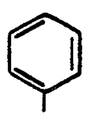 | 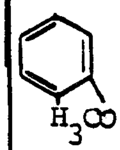 | - | $-CH_2-$ | - | Oxalat<br>108° C<br>Essigester |
| 88 | 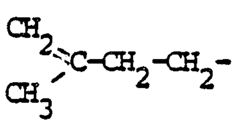 | 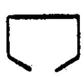 | 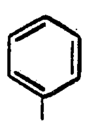 | 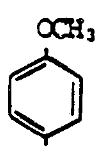 | - | $-CH_2-$ | - | Oxalat<br>99° C<br>Essigester |
| 89 | 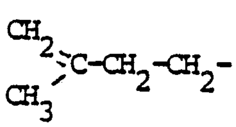 | 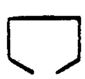 | 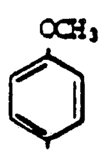 | 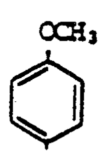 | - | $-(CH_2)_2-$ | - | Fumarat<br>105° C<br>Essigester |

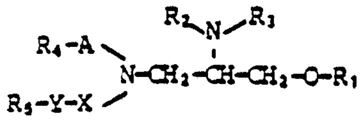

| Nr. | $R_1$ | $R_2$, $R_3$ | $R_4$ | $R_5$ | A | X | Y | |
|---|---|---|---|---|---|---|---|---|
| 90 | 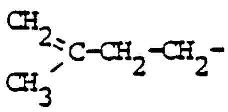 | 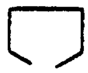 | 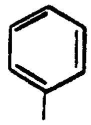 | 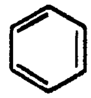 | - | - | - | Oxalat<br>104° C<br>Essigester |
| 91 | 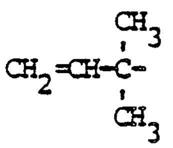 | 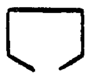 | 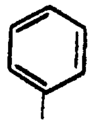 | 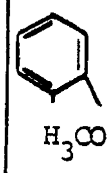 | - | $-CH_2-$ | - | Öl<br>m/e 408 |
| 92 | 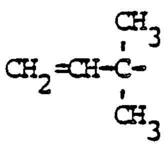 | 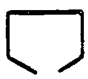 | 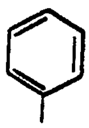 | 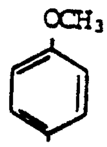 | - | $-CH_2-$ | - | Fumarat<br>148° C<br>Essigester |
| 93 | 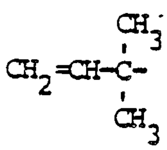 | 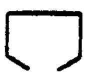 | 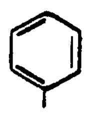 | 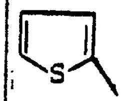 | - | $-CH_2-$ | - | Öl<br>m/e 384 |
| 94 | 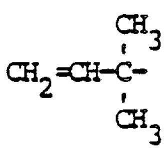 | 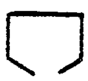 | 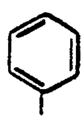 | 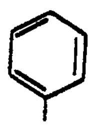 | - | - | - | Oxalat<br>135° C<br>Essigester |
| 95 | 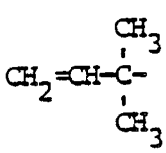 | 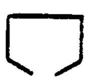 | 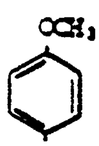 | 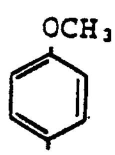 | - | $-CH_2-$ | - | Öl<br>m/e 438 |

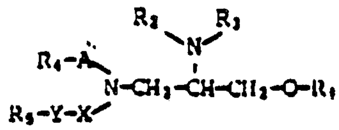

| Nr. | R₁ | R₂,R₃ | R₄ | R₅ | A | X | Y | |
|---|---|---|---|---|---|---|---|---|
| 96 | 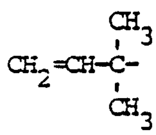 | 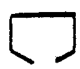 | 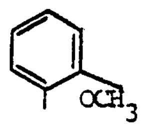 | 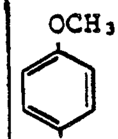 | - | $-CH_2-$ | - | Öl<br>m/e 438 |
| 97 | 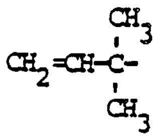 | 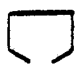 | 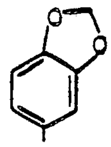 | 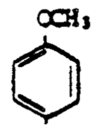 | - | $-CH_2-$ | - | Öl<br>m/e 452 |
| 98 | 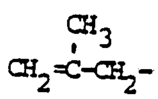 | 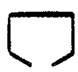 | 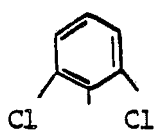 | 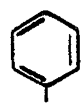 | - | $-CH_2-$ | - | Fumarat<br>101° C<br>Essigester |
| 99 | 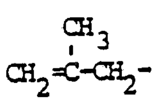 | 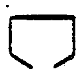 | 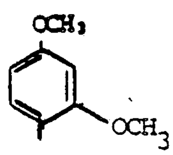 | 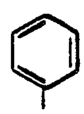 | - | $-CH_2-$ | - | Öl<br>m/e 424 |
| 100 | 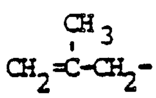 | 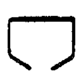 | 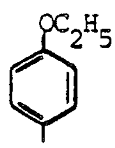 | 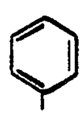 | - | $-CH_2-$ | - | Öl<br>m/e 408 |
| 101 | 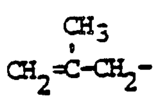 | 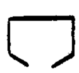 | 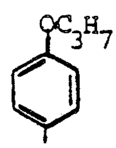 | 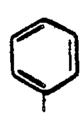 | - | $-CH_2-$ | - | Öl<br>m/e 422 |
| 102 | 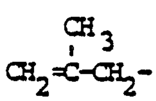 | 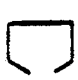 | 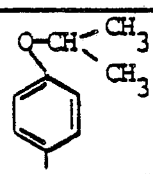 | 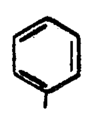 | - | $-CH_2-$ | - | Öl<br>m/e 422 |

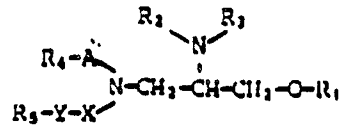

| Nr. | $R_1$ | $R_2,R_3$ | $R_4$ | $R_5$ | A | X | Y | |
|---|---|---|---|---|---|---|---|---|
| 103 | 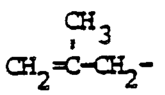 | 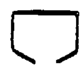 | 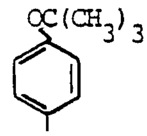 | 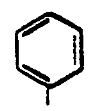 | - | $-CH_2-$ | - | Oxalat 128° C Essigester |
| 104 | 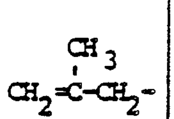 | 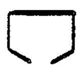 | 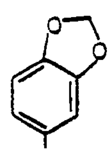 | 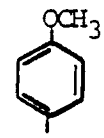 | - | $-CH_2-$ | - | Oxalat 103° C Essigester |

## Ansprüche

1. Verbindungen der allgemeinen Formel I

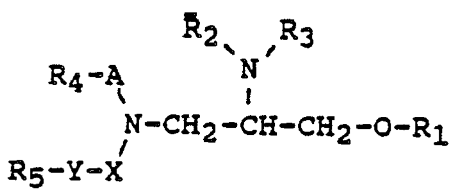

(I)

worin

$R_1$ einen geradkettigen oder verzweigten $C_1$-$C_{12}$ Alkylrest, der durch Phenyl-, Naphthyl- oder einen $C_3$-$C_7$-Cycloalkylrest substituiert sein kann,
einen geradkettigen oder verzweigten $C_2$-$C_6$-Alkenylrest, der durch einen $C_3$-$C_7$-Cycloalkylrest oder einen Phenyl- oder Naphthylrest substituiert sein kann,
einen $C_3$-$C_7$-Cycloalkylrest oder einen unsubstituierten oder einen ein- oder mehrfach substituierten mono- oder bicyclischen aromatischen Rest bedeutet,
wobei die Substituenten $C_1$-$C_4$ Alkyl, $C_1$-$C_4$ Alkoxy, Carboxyl oder Carbethoxy sein können,
$R_2$ und $R_3$ gleich oder verschieden sein können und einen geraden, verzweigten, gesättigten oder ungesättigten $C_1$-$C_6$-Alkylrest, der gegebenenfalls durch Hydroxy, $C_1$-$C_3$-Alkoxy oder $C_1$-$C_3$-Alkoxy-$C_1$-$C_3$ alkoxy substituiert sein kann, bedeuten oder zusammen mit dem Stickstoffatom einen gesättigten oder ungesättigten Ring bilden, der noch weitere Heteroatome enthalten kann und gegebenenfalls durch eine niedrige Alkylgruppe, eine niedrige Alkoxygruppe oder ein Sauerstoffatom substituiert ist,
A einen Valenzstrich oder einen geradkettigen oder verzweigten Alkylenrest mit 1-6 vorzugsweise 1-3 Kohlenstoffatomen bedeutet,
$R_4$ einen ein- oder mehrfach substituierten oder unsubstituierten mono- oder bicyclischen aromatischen oder heteroaromatischen Rest bedeutet,
wobei die Substituenten
Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-ethoxy-, Alkylamino-, Dialkylamino-, Alkoxy-carbonyl-ethyloxy-, Alkylmercapto-, Alkylsulfinyl-, Alkylsufonyl-, Alkylsulfonyloxy-, Carboxy-, Alkoxy-carbonyl-, Amino-carbonyl-, Mono- oder Dialkylamino-carbonyl-, Halogenalkyl- oder Cyano-gruppen sowie Halogenatome, wie Chlor, Brom oder Fluor bedeuten.

X einen Valenzstrich oder einen geradkettigen, verzweigten, gesättigten oder ungesättigten Kohlenwasserstoffrest von 1 - 6 Kohlenstofatomen bedeutet,

Y einen Valenzstrich oder ein Sauerstoffatom bedeutet, und

$R_5$ einen $C_3$-$C_7$-Cycloalkyl-Rest oder einen ein- oder mehrfach substituierten oder einen unsubstituierten mono- oder bicyclischen aromatischen oder heteroaromatischen Rest bedeutet,

wobei die Substituenten

Alkyl-, Alkoxy-, $C_2$-$C_6$-Alkenyloxy-, Aralkoxy-, Hydroxy-, Hydroxy-alkoxy-, Alkoxy-alkoxy-, Alkoxy-carbonyl-alkyloxy-, $C_1$-$C_2$-Alkylendioxy-, Dialkylamino-, Alkylmercapto-, Alkylsulfinyl-, Alkylsulfonyl-, Alkylsulfonyloxy-, Hydroxy-alkyl-, Carboxy-, Alkoxycarbonyl-, Amino-carbonyl-, Mono- oder Dialkylamino-carbonyl- oder Cyano-gruppen sowie Halogenatome, wie Chlor, Brom oder Fluor bedeuten

mit der Maßgabe, daß Y kein Sauerstoffatom darstellen darf, wenn

X einen Valenzstrich bedeutet

und daß, in dem Fall

daß $R_1$ einen gesättigten Kohlenwasserstoffrest darstellt, X einen Rest mit mindestens zwei Kohlenstoffatomen bedeuten muß,

sowie deren pharmakologisch verträgliche Salze und deren optische Isomere .

2. Verfahren zur Herstellung von Verbindungen der Formel I

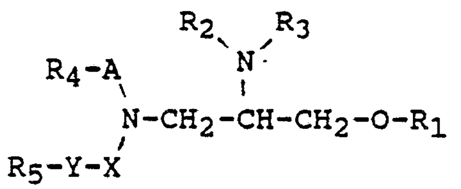

(I)

worin

$R_1$ einen geradkettigen oder verzweigten $C_1$-$C_{12}$ Alkylrest, der durch Phenyl-, Naphthyl- oder einen $C_3$-$C_7$-Cycloalkylrest substituiert sein kann,

einen geradkettigen oder verzweigten $C_2$-$C_6$-Alkenylrest, der durch einen $C_3$-$C_7$-Cycloalkylrest oder einen Phenyl- oder Naphthylrest substituiert sein kann,

einen $C_3$-$C_7$-Cycloalkylrest oder einen unsubstituierten oder einen ein- oder mehrfach substituierten mono- oder bicyclischen aromatischen Rest bedeutet,

wobei die Substituenten $C_1$-$C_4$ Alkyl, $C_1$-$C_4$ Alkoxy, Carboxyl oder Carbethoxy sein können,

$R_2$ und $R_3$ gleich oder verschieden sein können und einen geraden, verzweigten, gesättigten oder ungesättigten $C_1$-$C_6$-Alkylrest, der gegebenenfalls durch Hydroxy, $C_1$-$C_3$-Alkoxy oder $C_1$-$C_3$-Alkoxy-$C_1$-$C_3$ alkoxy substituiert sein kann, bedeuten oder zusammen mit dem Stickstoffatom einen gesättigten oder ungesättigten Ring bilden, der noch weitere Heteroatome enthalten kann und gegebenenfalls durch eine niedrige Alkylgruppe, eine niedrige Alkoxygruppe oder ein Sauerstoffatom substituiert ist,

A einen Valenzstrich oder einen geradkettigen oder verzweigten Alkylenrest mit 1-6 vorzugsweise 1-3 Kohlenstoffatomen bedeutet,

$R_4$ einen ein- oder mehrfach substituierten oder unsubstituierten mono- oder bicyclischen aromatischen oder heteroaromatischen Rest bedeutet,

wobei die Substituenten

Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-ethoxy-, Alkylamino-, Dialkylamino-, Alkoxy-carbonyl-ethyloxy-, Alkylmercapto-, Alkylsulfinyl-, Alkylsufonyl-, Alkylsulfonyloxy-, Carboxy-, Alkoxy-carbonyl-, Amino-carbonyl-, Mono- oder Dialkylamino-carbonyl-, Halogenalkyl- oder Cyano-gruppen sowie Halogenatome, wie Chlor, Brom oder Fluor bedeuten.

X einen Valenzstrich oder einen geradkettigen, verzweigten, gesättigten oder ungesättigten Kohlenwasserstoffrest von 1 - 6 Kohlenstofatomen bedeutet,

Y einen Valenzstrich oder ein Sauerstoffatom bedeutet, und

$R_5$ einen $C_3$-$C_7$-Cycloalkyl-Rest oder einen ein- oder mehrfach substituierten oder einen unsubstituierten mono- oder bicyclischen aromatischen oder heteroaromatischen Rest bedeutet,

wobei die Substituenten

Alkyl-, Alkoxy-, $C_2$-$C_6$-Alkenyloxy-, Aralkoxy-, Hydroxy-, Hydroxy-alkoxy-, Alkoxy-alkoxy-, Alkoxy-carbonyl-alkyloxy-, $C_1$-$C_2$ Alkylendioxy-, Dialkylamino-, Alkylmercapto-, Alkylsulfinyl-, Alkylsulfonyl-, Alkylsulfonyloxy-, Hydroxy-alkyl-, Carboxy-, Alkoxycarbonyl-, Amino-carbonyl-, Mono- oder Dialkylamino-carbonyl- oder Cyano-gruppen sowie Halogenatome, wie Chlor, Brom oder Fluor bedeuten

mit der Maßgabe, daß Y kein Sauerstoffatom darstellen darf, wenn
X einen Valenzstrich bedeutet
und daß, in dem Fall
daß $R_1$ einen gesättigten Kohlenwasserstoffrest darstellt, X einen Rest mit mindestens zwei Kohlenstoffatomen bedeuten muß,
sowie deren pharmakologisch verträglichen Salze und deren optische Isomere,
dadurch gekennzeichnet, daß man in üblicher Weise
a) eine Verbindung der allgemeinen Formel II,

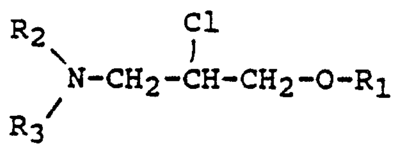

(II)

in der $R_1$, $R_2$, und $R_3$ die oben genannten Bedeutungen besitzen, mit einer Verbindung der allgemeinen Formel III,

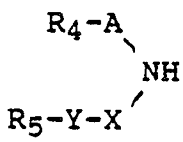

(III)

in der A, X, Y, $R_4$ und $R_5$ die oben genannten Bedeutungen besitzen, umsetzt
oder
b) eine Verbindung der allgemeinen Formel IV,

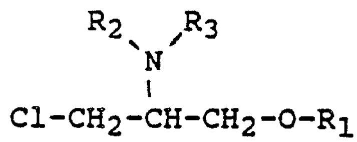

(IV)

in der $R_1$, $R_2$, und $R_3$ die oben genannten Bedeutungen besitzen, mit einer Verbindung der allgemeinen Formel III umsetzt,
oder
c) eine Verbindung der allgemeinen Formel V,

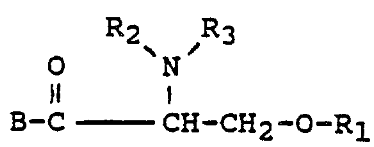

(V)

in der $R_1$, $R_2$, und $R_3$ die oben genannten Bedeutungen besitzen und B ein Halogenatom oder eine Alkoxygruppe bedeutet, einer Amidbildungsreaktion mit einer Verbindung der allgemeinen Formel III unterwirft und die erhaltene Verbindung der allgemeinen Formel VI

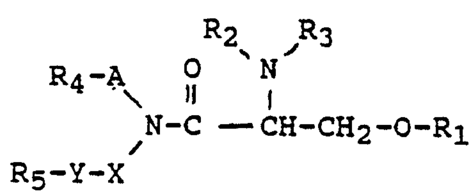

(VI)

in der $R_1$, $R_2$, $R_3$, $R_4$, $R_5$, A, X und Y die oben genannten Bedeutungen besitzen, einer Reduktion mit einem komplexen Hydrid oder Diboran unterzieht,
und anschließend gewünschtenfalls die erhaltenen Verbindungen in ihre pharmakologisch verträglichen

Salze überführt.

3. Arzneimittel, enthaltend eine Verbindung gemäß Anspruch 1 neben üblichen Träger- und Hilfsstoffen.

4. Verwendung von Verbindungen gemäß Anspruch 1 zur Behandlung von Herz- und Kreislauferkrankungen.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung: EP 88112675.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 138 684 (RIOM LABORATOIRES-C.E.R.M.)<br>* Ansprüche; Seite 2, Zeile 26 - Seite 3, Zeile 36 *<br>-- | 1-3 | C 07 D 295/12<br>C 07 D 333/20<br>C 07 D 207/44<br>C 07 D 317/66<br>C 07 D 307/78<br>C 07 D 317/58<br>C 07 D 307/52<br>A 61 K 31/40<br>A 61 K 31/13<br>C 07 C 93/02<br>A 61 K 31/44 |
| A | WO - A1 - 83/02 274 (CARTER-WALLACE)<br>* Ansprüche; Seite 1, Zeile 18 *<br>-- | 1,2 | |
| P,A | EP - A1 - 0 237 191 (MCNEILAB)<br>* Ansprüche *<br>-- | 1-3 | |
| D,A | DE - A1 - 2 802 864 (CENTRE EUROPEEN DE RECHERCHES MAUVERNAY)<br>* Ansprüche *<br>---- | 1-3 | |
| | | | **RECHERCHIERTE SACHGEBIETE (Int. Cl.4)**<br>C 07 D 295/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3

Unvollständig recherchierte Patentansprüche: -

Nicht recherchierte Patentansprüche: 4

Grund für die Beschränkung der Recherche:

(Verfahren zur therapeutischen Behandlung des menschlichen oder tierischen Körpers, Art. 52 (4) EPÜ)

| Recherchenort | Abschlußdatum der Recherche | Prüfer |
|---|---|---|
| WIEN | 14-10-1988 | KÖRBER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument